# EUROPEAN PATENT SPECIFICATION

(11) **EP 0 498 920 B1**
(45) Date of publication and mention of the grant of the patent: **26.01.2000**
(21) Application number: 91107960.6
(22) Date of filing: 16.05.1991
(51) Int. Cl.: G01N 33/569, C12Q 1/04, G01N 33/543

(54) **Assay method for detecting the presence of bacteria**
Testverfahren zum Nachweis der Anwesenheit von Bakterien
Méthode d'essai pour détecter la présence de bactéries

(30) Priority: 14.02.1991 US 654967
(43) Date of publication of application: 19.08.1992
(73) Proprietor: VICAM, L.P., Sommerville, Massachusetts 02145 (US)
(72) Inventor: Benjamin, Thomas L., Cambridge, MA. 02139 (US); Chen-Wu, Joan, Brookline, MA. 02146 (US); Hansen, Thomsen, Brookline, MA 02146 (US); Jackson, Barbara, Roslindale, MA 02131 (US); Livingstone, David, Brookline, MA.02146 (US); Tannenbaum, Steven, Framingham, MA. 01701 (US); Wogan, Gerald, Belmont, MA 02178 (US)
(74) Representative: VOSSIUS & PARTNER

(56) References cited:
- WO-A-89/01162
- APPLIED AND ENVIRONMENTAL MICROBIOLOGY vol. 56, no. 11, 1 November 1990, WASHINGTON DC USA pages 3478 - 3481 E. SKJERVE ET AL. 'Detection of Listeria monocytogenes in foods by immunomagnetic separation.'
- EPPO BULLETIN vol. 17, 1978, WAGENINGEN NL pages 139 - 148 W.L. VAN VUURDE 'New approach in detecting phytopathogenic bacteria by combined immunoisolation and immunoidentification assays.'

## Description

This invention relates to an assay method for quickly and easily detecting the presence of bacteria. In particular, an immunoassay method is utilized to detect the presence of viable bacteria strains in foods and other potentially contaminated samples using an assay characterized by 1) capture of specific bacterial cells with specific antibodies; (2) incubation of the captured cells to form bacteria colonies; (3) imprint of the colonies to a colony lift membrane; and (4) immunochemical detection and species identification of the colonies on the colony lift membrane.

The presence of bacterial pathogens is a well recognized cause of severe illness, so that there is an ever present need for the detection of such pathogens in both clinical specimens (i.e. blood, tissue, urine and other body extracts and fluids), agricultural specimens (such as food products) and environmental specimens (such as surfaces in food processing plants).

However, current tests for the detection of bacterial pathogens, such as in food, typically require a number of days to complete. During this period of time, between sampling and assay determination, fresh food and dairy products will enter the food chain and therefore be consumed by the public. If a test indicates the presence of pathogens, expensive product recalls may result, or, worse, before the test results are discovered an outbreak of sickness may occur.

As stated above, traditional methods to detect the presence of bacterial food pathogens require an extended period of time, basically due to the need for an enrichment/incubation period. This incubation/enrichment period is intended to allow for recovery of injured bacteria, growth of these bacteria from a background of competing microorganisms and an increase in bacterial cell numbers to more readily aid in identification. In many cases a series of two or three separate incubations is needed to isolate the target bacteria. However, such enrichment steps can actually compromise test sensitivity by killing some of the cells sought to be measured.

In the standard FDA procedure for detection of Listeria in food products (Bacteriological Analytical Manual, 6th ed., 1984; Supplement, September 1987, Chapter 29) 25 g or 25 ml of a food sample is mixed with 225 ml of enrichment broth. This sample in broth mixture is incubated for 7 days. At the end of days 1 and 7 a sample of the broth culture is streaked onto petri plates containing selective growth agar and these plates are incubated for an additional 2 days. Identification of Listeria colonies by eye confirms the presence of Listeria in the original food sample. This identification, however, is subjective, and therefore prone to misinterpretation, and this procedure requires a minimum of 7 days to confirm Listeria negative samples.

More recent methods of bacterial detection in food products have utilized immunoassays. Antibodies to an antigen present in the bacteria of interest are generally used in these methods in some form of a two site assay. That is, one antibody is immobilized and acts to capture the target bacterial antigens. This allows for separation of the target antigen from the food sample. A second antibody to this antigen (having the same or a different epitope) is labeled in some fashion such as radioactively with ¹²⁵I or enzymatically with horse radish peroxidase, and when added to the immobilized antibody antigen complex also becomes immobilized. Subsequent steps remove unbound labeled antibody. The label left attached is measured and usually compared against standards (positive and negative controls) to determine the presence of the target bacteria. At least one of the two antibodies used in the two site assay must be specific to the target bacteria. This type of immunoassay is known as a direct assay. Other forms, such as the competition assay are also used but tend to be less sensitive, and technically more difficult to perform. Immunoassays are described in Skjerve, E. et al. Applied and Environmental Microbiology, Vol 56, No. 11 (1990), 3478 to 3481 and in WO 89/01162. Skjerve et al. describe a method for detection of Listeria in foods by immunomagnetic separation. The detection of the bacteria captured on the immunomagnetic beads is by spreading the beads on agar and visually detecting the colonies. The document reports a sensitivity of detection of 100 to 200 bacterial cells per ml. WO 89/01162 describes a method for detecting microorganisms in a sample, comprising exposing the sample to a solid support to which antibodies specific for the organism are adsorbed and growing the bound organism to a detectable level followed by immunoassay or releasing the organism from the support and growing them to visually detectable colonies.

Because of the actual sensitivity limit of these assays it remains necessary to culture the target bacteria from the food sample. In some of the newer immunoassay tests incubation times have been reduced and the number of separate incubation steps have also been reduced. The resulting tests, however, still require about 48 hours to complete.

In a generalized "rapid" immunoassay for detection of bacteria, including Listeria, 25 g or 25 ml of food sample is mixed with 225 ml of enrichment broth, as in the FDA procedure. This culture mixture is incubated for 24 hours. From this 250 ml of culture, 1 ml is removed and added to 9 ml of selective growth medium. This selective culture mixture is incubated for an additional approximately 24 hours. At this point, depending upon the actual immunoassay format, some fraction of the total 10 ml subculture (usually 0.2 ml - 1.0 ml) is tested by immunoassay for the presence of Listeria.

In summary, these existing "rapid" immunoassay procedures for bacterial detection in food samples all require at least one (usually two or more) dilution of sample (into growth medium) followed by an assay procedure which only utilizes a fraction of this final culture. The actual assay sample thus only corresponds to a small fraction of the original food sample. The bacterial culture step, or steps, must therefore overcome this dilution factor, adding to the amount of needed culture time. In addition, the utilized enrichment steps may kill the bacteria sought to be identified, producing a high false negative rate.

These and other disadvantages of the prior art methods are overcome by the present invention which provides a fast and accurate method for the detection of viable bacteria in various possible samples.

It is therefore one object of the present invention to provide a method for quickly detecting the presence of bacteria, including potentially pathogenic bacteria.

It is another object of the invention to provide a method for the detection of viable bacteria which minimizes or eliminates the selective culturing steps.

It is a further object of the invention to provide a method for the detection of bacteria by which the detection can be easily made by viewing with the human eye.

These and other objects of the invention are accomplished by providing a method for detecting the presence of bacteria which comprises:
(a) combining a sample to be tested for the presence of selected bacteria with magnetic beads having immobilized thereon first antibodies to said selected bacteria;
(b) incubating said sample and said magnetic beads having said antibodies immobilized thereon to thereby capture cells of said selected bacteria from said sample;
(c) exposing to a magnetic field said magnetic beads having bound thereto said first antibodies and captured bacteria cells to thereby separate said magnetic beads from said sample;
(d) culturing said magnetic beads and captured bacteria cells on a solid medium to form colonies of said bacteria;
(e) contacting said colonies with a colony lift membrane whereby colony material from said colonies adheres to said membrane; and
(f) contacting said membrane with detector antibodies which bind to said colony material of said selected bacteria and the presence of said detector antibody on said membrane is detected to provide visual evidence of the presence on said membrane of colony material from said selected bacteria.

As noted above, one object of the invention is to provide an assay procedure for rapid and easy identification of the presence of a particular bacteria of interest in a sample. The assay procedure is applicable to the detection of bacteria, but may also be applied to the detection of molds and yeast which are not included within the present invention. Of particular importance is the detection of the presence of potentially harmful contaminants, particularly those which cannot be visually detected by eye. A broad range of contaminants can be detected by the assay, so long as the contaminant can be cultured to form colonies and antibodies can be raised against the contaminant.

The assay is used to detect various bacteria, and can be utilized to detect the presence of any specific, selected bacteria of interest. The bacteria can be either pathogenic or non-pathogenic, although the invention is particularly important for detection of potentially contaminating pathogenic bacteria. Specific bacteria detectable by the assay of the invention include, for example, Listeria, Campylobacter, Escherichia coli, Salmonella, Clostridia (such as Clostridium botulinum and Clostridium perfingens), Shigella, Staphylococci (such as Stapylococcus aureus), Vibrio (such as Vibrio vulnificus, Vibrio cholerae and Vibrio parahaemolyticus), Yersinia (such as Yersinia enterocolitica and Yersinia pseudotuberculosis) Plesiomonas shigelloides, Bacilli (such as Bacillus cereus) and Aeromonas (such as Aeromonas hydrophila).

In addition, various molds can be detected, including Byssochlamys, Fusarium, Geotrichum, Penicillium and Scopulariopsis and various yeasts can be detected, such as Kluyveromyces, Pichia, Saccharomyces, Candida and Rhodotorula, but are not subject of the present invention.

The method of the invention, therefore, generally comprises the following procedural steps:
1) The sample to be tested is, if necessary, liquified or otherwise prepared for use in the assay.
2) The liquid sample is combined with a solid matrix which consists of magnetic beads having immobilized thereon monoclonal or polyclonal antibodies to the selected bacteria of interest, the presence of which is to be determined by the assay. If present, the target selected bacteria cells are thereby immobilized onto the solid matrix. The solid matrix with attached immobilized bacteria is then washed to remove any remaining sample.
3) The solid matrix with the immobilized bacteria are then placed on a culture medium, and the bacteria are allowed to grow to form colonies.
4) The thus formed bacterial colonies are then contacted with a colony lift membrane, and the membrane is removed, whereby the colony material from the bacterial colonies is attached to the lift membrane.
5) The membrane is treated with a fixing agent to fix the colony material to the membrane and the membrane is then blocked to lower non-specific reactivity.
6) The membrane, having fixed thereto material from the bacterial colonies, is then treated with a labeled or unlabeled first antibody specific to the bacteria of interest to be detected, and the membrane is washed to remove any unbound first antibody.
7) If the first antibody was unlabeled, the membrane is then treated with a second antibody which is labeled and is specific for the first antibody, and the membrane is washed to remove any unbound labeled second antibody.
8) The presence of labeled first or second antibody on the membrane is then detected, specifically by means to permit visual evidence of the presence of colonies of the bacteria of interest.

As can be seen from the above method scheme, the method of the invention is particularly characterized by the use of an antibody/s to first select out bacteria from a sample. The antibody/s used for this step need not be totally specific to the bacteria of interest as subsequent selective and specific steps combine to confer the ultimate specificity of the assay for only specific bacteria of interest. Further uniquely characterizing the method of the invention is the use of a colony lift membrane and subsequent detection steps. Using these characteristic features, the method of the invention can be importantly applied to the rapid and specific detection of viable strains of bacteria, particularly detection by a simple visual means, detectable by the human eye. The assay particularly provides a very sensitive assay detection, capable of detecting 1 colony forming unit (CFU) per 25 ml of sample.

### Step 1: Liquefaction

The assay of the invention can be utilized to detect the presence of bacteria in a wide variety of samples, both solid and liquid, including food, agricultural products, environmental samples and various clinical specimens. If the sample is liquid, it can be per se subjected to the procedure of the invention, or first diluted, or concentrated by centrifugation. On the other hand, if the sample is solid, it should be first liquified (for example in water) using standard known techniques, such as by use of a blender/stomacher. The liquid or liquified sample may, if desired, be filtered through a course paper, glass or other matrix filter to remove particulates. If the sample to be tested is an environmental sample, then swabs or scrapings of the tested surface or material are mixed in a collection buffer and then treated as a liquified food sample.

### Step 2: Immobilization With Antibodies

Immobilized antibodies (polyclonal or monoclonal) to target bacterial cells are used to separate the bacteria cells from the sample. In this step, one or more antibodies may be used to recognize all target strains of the bacteria genus of interest.

The antibodies used in this step recognize bacteria cell surface antigens, and these antibodies are preferably immobilized on the microspheres which can be mixed with the sample and separated from the sample mixture in some manner subsequent to capture of the target bacterial cells. The microspheres used as the immobilizing matrix are magnetic beads. After an incubation period in which the liquid sample or liquified sample is mixed with the magnetic beads having antibodies attached, the antibody bound particles and attached bacteria are then separated from the sample by means of a magnetic field (magnetic capture) and washed to remove other potential impurities.

As the antibodies for capture, and, as discussed later, for detection, of the bacteria, any class of antibodies can be used (including IgG and IgM) and either polyclonal antibodies or monoclonal antibodies can be used depending upon various factors, including the degree of sensitivity desired. If polyclonal antibodies are to be used, then such antibodies can be prepared according to per se known procedures. For example, procedures such as those described by Hurn, B.A. et al. (1980) in Meth. in Enzymology, Ed. Van Vanakis, H. and Langone, J., pp. 104-142, can be used.

The preparation of monoclonal antibodies is known and if monoclonal antibodies are to be used in this invention, they are prepared using the method originally authored by Milstein and Köhler and published in Nature (1975), 256, pps. 495-497. The basic process involves injecting an animal, usually a mouse, with an immunogenic substance. After suitable time for antibody production to the immunogen, the mouse is sacrificed. Cells are removed from the spleen and fused with myeloma cells. Hybridoma cells resulting from this fusion are able to reproduce in vitro, and each expresses genetic information for one specific antibody. The antibodies produced from one hybridoma fusion thus will only recognize a single antigenic determinant of the immunogen.

Cells cultured from individual hybridoma cells are screened for production of antibodies to the target antigenic determinant. Those hybridomas positive for the target antigen are further screened to identify those having any affinity. The monoclonal antibodies used in the present invention should have an affinity of at least 10⁸ liters/mole. Monoclonal antibodies displaying all of these characteristics are then screened using actual assay conditions to determine if the assay condition alters the antibody binding characteristics or affinity, and to screen out those with cross reactivity to possible contaminating antigens.

The antibodies are immobilized on magnetic beads. This can be accomplished by procedures which are per se known, such as those described in U.S. Patent No. 3,970,518; No. 4,018,886, No. 4,855,045 and No. 4,230,685. In one embodiment, attachment of antibodies to magnetic particles is accomplished through a Protein A intermediate. That is, Protein A is first attached to the magnetic particles and the antibodies of choice are then bound to the Protein A. The use of the Protein A intermediate greatly increases the effectiveness of capture by the attached antibodies. (Forsgren et al. (1977) J. Immunol. 99:19) Protein A attaches to the Fc portion of IgG subclass antibodies, thus extending and presenting the Fab portion of these antibodies. The resulting correct orientation of the antibodies and extension away from the particles leads to a very effective interaction between the bound antibodies and their target.

The method of attachment of Protein A to magnetic particles may proceed by any of several processes available through the scientific literature. In one such procedure, magnetic iron oxide particles of approximately 1 micrometer diameter are chemically derivatized by reaction, first with 3-aminopropyltriethoxysilane, then with glutaraldehyde. The derivatized magnetic particles are then mixed with Protein A resulting in a magnetic particle to which Protein A is covalently attached. The antibodies are then added to the Protein A magnetic particles and after a short incubation the protein A-antibody complexes form. (Weetall, H.H. (1976) Meth. in Enzymol. 44:134-148) These derivatized particles with Protein A-antibodies attached are now ready for use in bacterial cell capture.

### Step 3: Growth of Bacterial Colonies

The captured and immobilized bacterial cells are placed on a medium on which the cells will grow and are incubated. Incubation is conducted for a time sufficient to form bacterial colonies visible to the eye.

Particular media for incubation depend, of course, upon the bacteria of interest to be detected. Such mediums, preferably solid, are per se known to those skilled in the art for various bacteria, as disclosed, for example, in T. Maniatis et al., Molecular Cloning - A Laboratory Manual, Cold Spring Harbor Lab. 1982. Incubation times and conditions are also varied and per se known, depending upon the particular bacteria of interest. Generally, sufficient growth is accomplished within 6 to 24 hours.

### Step 4: Colony Lift onto Membranes

After incubation of the bacteria is completed, a colony lift membrane is placed in contact with the growth medium, whereby the colonies attach and imprint colony material to the membrane. Colony lift membranes are per se known and may be comprised of, for example, nitrocellulose or nylon. The membrane is preferably cut to the size of the container or dish containing the growth medium, so that all colonies growing on the container are overlayed with the same single sheet. In this manner, the sheet/membrane acquires the same pattern of colonies that was originally contained on the growth medium. The growth medium can be maintained and utilized for multiple colony lifts from the same plate, and the lift membranes can be utilized to replicate the same colonies onto additional growth medium plates ("replica plating").

### Step 5: Fixing of the Colonies to the Lift Membrane

It is then necessary to fix the colony material to the lift membrane by treatment with an appropriate fixing agent in order to ensure that the colonies adhere more firmly to the sheet. Suitable fixing treatments include placing the lift membrane in a solution of methanol and placing the membrane in a solution of the detergent sodium dodecyl sulfate with brief heating to 70°C.

It may also be desirable to treat the fixed colonies and membrane with per se known blocking agents to prevent non-specific reactivity of the subsequent detecting antibodies. Suitable blocking agents include, for example, caseine and BSA.

### Step 6: Treatment with First Bacteria Specific Antibody

The lift membrane having fixed thereto the colony material from the bacterial colonies is next contacted with antibodies specific to the bacteria of interest. As noted above, either polyclonal or monoclonal antibodies can be utilized, but in either case have affinity for the particular bacteria to be detected. These antibodies, when contacted with the lift membrane, will adhere/bind to material from the specific target bacteria colonies, but will not bind to the other colonies.

Useful polyclonal antibodies include, for example, those from Difo poly sera raised in rabbits. These antibodies can also be specific for particular strains to be detected. For example, Listeria monocytogenes includes strains SV 1/2b, 1/2c, 3a, 3b, 3c, 4a, 4a/b, 4b, 4c, 4d, 4e, 7, with strains SV 1/2a, 1/2b and 4b being the most common pathogenic strains. It is useful, therefore, to detect the presence in a sample of pathogenic Listeria by utilizing a polyclonal seria against Listeria serovars 1/2 and 4.

After allowing time for binding of the antibodies, the membrane sheet is washed to remove any antibody which is not specifically bound to material from the bacteria colonies of interest.

### Steps 7 and 8: Treatment of Labelled Antibody and Detection

Treatment Step 6 with a first antibody specific to the bacteria cells of interest provides the first step for selection and identification of the specific bacteria of interest. The membrane sheet is then contacted with a second antibody. Again, this antibody may be either polyclonal or monoclonal, but importantly is (a) capable of binding to the first antibody and (b) labeled in a manner to enable subsequent detection.

If the first antibody is, for example, the above-noted Difco poly sera, then the second antibody is an anti-rabbit IgG/label conjugate. If the first antibody is a monoclonal antibody derived from mouse, than the second antibody is an anti-mouse IgG/label conjugate.

Alternatively, the antibody utilized in Step 6 can itself be a labeled antibody. The assay can then proceed to detection of the label (Step 8) without the need for use of a second antibody.

With respect to labeling of the antibodies, these are labeled either directly or indirectly with labels used in other known immunoassays. Direct labels may include fluorescent, chemiluminescent, bioluminescent, radioactive, metallic, biotin or enzymatic molecules. Methods of combining these labels to antibodies or other macromolecules are well known to those in the art. Examples include the methods of Hijmans, W. et al. (1969), Clin. Exp. Immunol. 4, 457-, for fluorescein isothiocyanate, the method of Goding, J.W. (1976), J. Immunol. Meth. 13, 215-, for tetramethylrhodamine isothiocyanate, and the method of Ingrall, E. (1980), Meth. in Enzymol. 70, 419-439 for enzymes.

These detector antibodies may also be labeled indirectly. In this case the actual detection molecule is attached to a secondary antibody or other molecule with binding affinity for the anti-bacteria cell surface antibody. If a secondary antibody is used it is preferably a general antibody to a class of antibody from the animal species used to raise the anti-bacteria cell surface antibodies.

For example, the second antibody may be conjugated to an enzyme, either alkaline phosphatase or to peroxidase. To detect the label, after the membrane sheet is contacted with the second antibody and washed, the membrane sheet is immersed in a solution containing a chromogenic substrate for either alkaline phosphatase or peroxidase. A chromogenic substrate is a compound which can be cleaved by an enzyme to result in the production of some type of detectable signal which only appears when the substrate is cleaved from the base molecule. The chromogenic substrate is colorless, until it reacts with the enzyme, at which time an intensely colored product is made. Thus, material from the bacteria colonies adhered to the membrane sheet will become an intense blue/purple/black color, or brown/red while material from other colonies will remain colorless. Examples of detection molecules include fluorescent substances, such as 4-methylumbelliferyl phosphate, and chromogenic substances, such as 4-nitrophenylphosphate, 3,3',5,5'-tetramethylbenzidine and 2,2'-azino-di-[3-ethelbenz-thiazoliane sulfonate (6)]. In addition to alkaline phosphatase and peroxidase, other useful enzymes include β-galactosidase, β-glucuronidase, α-glucosidase, β-glucosidase, α-mannosidase, galactoseoxidase, glucose oxidase and hexokinase.

### Listeria Detection Example

The following is a specific example to show the use of the assay of the invention to particularly detect the presence of Listeria, Salmonella or E. coli.
1. A food sample is liquified and combined with magnetic beads having immobilized thereon antibodies to Listeria.
2. After an incubation time of about 30 minutes to 2 hours, the mixture is subjected to magnetic capture to remove the magnetic beads having bacterial cells immobilized thereon and the beads are washed to remove undesirable impurities and unbound material or cells.
3. The separated magnetic beads are placed on the surface of an appropriate bacterial growth medium in a Petri dish.
4. After an incubation period of time ranging from 6-24 hours, place a colony lift membrane (e.g. Bio-Rad cat. #162-0164) onto the plate of solid growth medium to obtain an imprint of the colonies on the membrane. Wait 5 minutes before peeling the membrane from the plate. The plate may be stored at 4°C, for use in future testing.
5. Place the colony lift membrane, colony side up, into a Petri dish containing 10ml methanol for 5 minutes, to kill bacteria and to fix antigens onto the membrane surface so that they will not wash off.
6. Wash the membrane with PBS (phosphate buffered saline)/0.01% merthiolate for 2-5 minutes. Remove wash fluid and repeat wash two additional times, for a total of three washes.
7. Block the membrane to prevent non-specific reactivity of the detecting antibodies by placing the membrane in 6ml of 2% non-fat dry milk/1% horse serum, PBS/0.01% merthiolate and shake on a gyrotary shaker for 30 minutes.
8. Wash the membrane with PBS/0.05% Tween-20 for 2-5 minutes, for a total of three washes.
9. Incubate the membrane with 6 ml of first antibody, capable of binding to the bacteria of interest. The first antibody should be diluted in PBS/0.05% Tween to a good working dilution which will vary with the antibody used, and incubated with shaking of the membrane for 30 minutes.
10. Wash the membrane with PBS/0.05% Tween-20 for 2-5 minutes, for a total of two washes.
11. Incubate the membrane with 6 ml of second antibody, a goat anti-rabbit or goat anti-mouse IgG and IgM antibody conjugated to peroxidase or alkaline phosphatase. (This means the second antibodies are produced by a goat that was injected with rabbit IgG and IgM, or with mouse IgG and IgM, and the second antibodies recognize the rabbit and mouse antibodies and react with them.) These second antibodies are chemically bonded to the enzyme peroxidase, so that when a substrate for that enzyme is present, areas where the second antibody is present too will become colored. The second antibody attaches to the first antibody, which is specific for the bacteria, so that color is produced on the colony lift membrane at places where material from colonies of bacteria are present. The incubation should be in PBS/0.05% Tween 20 for 30 minutes or more, with shaking.
12. Wash the membrane with PBS/0.05% Tween-20 for 2-5 minutes, for a total of two washes.
13. Wash the membrane with water for 2-5 minutes, for a total of two washes.
14. Incubate the membrane with 6ml of fresh peroxidase or alkaline phosphatase substrate solution, with gentle shaking until brown or blue dots of colony material appear, or until the background where no colonies are present becomes pale tan or pale blue. A typical incubation requires about 5 minutes.
15. Stop the reaction by washing the membrane with water for 2-5 minutes, for a total of two washes.
16. The presence of Listeria, Salmonella or E. coli cells is then indicated by color formation on the colony material adhered to the membrane sheet.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A method for detecting the presence of bacteria which comprises:
(a) combining a sample to be tested for the presence of selected bacteria with magnetic beads having immobilized thereon first antibodies to said selected bacteria;
(b) incubating said sample and said magnetic beads having said antibodies immobilized thereon to thereby capture cells of said selected bacteria from said sample;
(c) exposing to a magnetic field said magnetic beads having bound thereto said first antibodies and captured bacteria cells to thereby separate said magnetic beads from said sample;
(d) culturing said magnetic beads and captured bacteria cells on a solid medium to form colonies of said bacteria;
(e) contacting said colonies with a colony lift membrane whereby colony material from said colonies adheres to said membrane; and (f) contacting said membrane with detector antibodies which bind to said colony material of said selected bacteria and the presence of said detector antibody on said membrane is detected to provide visual evidence of the presence on said membrane of colony material from said selected bacteria.

2. The method according to claim 1, wherein said membrane having colony material adhered thereto is contacted with second antibodies specific for said selected bacteria, whereby said second antibodies bind to said colony material from said colonies of said selected bacteria; said membrane is subsequently contacted with labeled antibodies specific for said second antibodies, and the presence of said label on said membrane is detected to thereby detect the presence of said selected bacteria in said sample.

3. The method according to claim 1, wherein said membrane is contacted with labeled antibodies specific for said selected bacteria, whereby said labeled antibodies bind to said colony material from said selected bacteria, and the presence of said label on said membrane is detected to thereby detect the presence of said selected bacteria in said sample.

4. The method according to claim 2 or 3, wherein said label is selected from fluorescent, radioactive, chemiluminescent, bioluminescent, and enzyme substrate molecules.

5. The method according to any one of the previous claims, wherein said first antibodies are polyclonal antibodies to said selected bacteria.

6. The method according to any one of claims 1 to 5, wherein said detector antibodies, second antibodies and labeled antibodies are, or are derived from monoclonal antibodies.

7. The method according to any one of claims 2 to 6, wherein said label is an enzymatic molecule, which when treated, produces a color change to provide visual evidence of the presence on said membrane of said label and colonies of said selected bacteria.

8. The method according to claim 7, wherein said enzymatic molecule is peroxidase, alkaline phosphatase, β-galactosidase, β-glucuronidase, α-glucosidase, β-glucosidase, α-mannosidase, galactose oxidase, glucoseoxidase, or hexokinase.

9. The method according to claim 8, wherein said membrane is treated with a chromogenic substance for said enzymatic molecule.

10. The method according to any one of the previous claims, wherein said colony lift membrane is comprised of nitrocellulose or nylon.

11. The method according to any one of the previous claims, wherein after contacting with said bacteria colonies, said membrane is treated with a fixing agent to fix said colonies to said membrane.

12. The method according to any one of the previous claims wherein said bacteria are selected from Listeria, Campylobacter, Escherichia coli, Salmonella, Clostridia, Shigella, Staphylococci, Vibrio, Yersinia, Plesiomonas strigelloides, Bacilli and Aeromonas.

13. The method according to claim 12, wherein said selected bacteria are Listeria.

14. The method according to claim 13, wherein said selected bacteria are pathogenic strains of Listeria.

## Patentansprüche

1. Verfahren zum Nachweis der Gegenwart von Bakterien, das folgende Schritte umfaßt:
(a) Zusammenbringen einer Probe, die auf das Vorhandensein von ausgewählten Bakterien zu testen ist, mit magnetischen Kügelchen, auf denen Erstantikörper gegen die ausgewählten Bakterien immobilisiert sind;
(b) Inkubation der Probe und der magnetischen Kügelchen, auf denen die Antikörper immobilisiert sind, um dadurch die Zellen der ausgewählten Bakterien aus der Probe einzufangen;
(c) Aussetzen der magnetischen Kügelchen, an die die Erstantikörper und die eingefangenen Bakterienzellen gebunden sind, einem magnetischen Feld, um damit die magnetischen Kügelchen von der Probe abzutrennen;
(d) Züchten der magnetischen Kügelchen und der eingefangenen Bakterienzellen auf einem festen Medium, um Kolonien der Bakterien zu bilden;
(e) Inkontaktbringen der Kolonien mit einer Kolonie-Hub-Membran, wobei Koloniematerial dieser Kolonien an der Membran anhaftet; und
(f) Inkontaktbringen der Membran mit Nachweisantikörpern, die an das Koloniematerial der ausgewählten Bakterien binden, und Nachweis der Gegenwart des Nachweisantikörpers auf der Membran, um einen sichtbaren Beweis für die Gegenwart des Koloniematerials der ausgewählten Bakterien auf der Membran zu liefern.

2. Verfahren nach Anspruch 1, wobei die Membran, an der Koloniematerial anhaftet, mit Zweitantikörpern in Kontakt gebracht wird, die spezifisch für die ausgewählten Bakterien sind, wobei die Zweitantikörper an das Koloniematerial von den Kolonien der ausgewählten Bakterien binden, die Membran anschließend mit markierten Antikörpern in Kontakt gebracht wird, die spezifisch für die Zweitantikörper sind, und die Gegenwart der Markierung auf der Membran nachgewiesen wird, um damit das Vorhandensein der ausgewählten Bakterien in der Probe nachzuweisen.

3. Verfahren nach Anspruch 1, wobei die Membran mit markierten Antikörpern in Kontakt gebracht wird, die spezifisch für die ausgewählten Bakterien sind, wobei die markierten Antikörper an das Koloniematerial der ausgewählten Bakterien binden, und die Gegenart der Markierung auf der Membran nachgewiesen wird, um damit die Gegenwart der ausgewählten Bakterien in der Probe nachzuweisen.

4. Verfahren nach Anspruch 2 oder 3, wobei die Markierung ein fluoreszierendes, radioaktives, chemilumineszierendes, biolumineszierendes oder Enzym-Substrat-Molekül ist.

5. Verfahren nach einem der vorstehenden Ansprüche, wobei die Erstantikörper polyclonale Antikörper gegen die ausgewählten Bakterien sind.

6. Verfahren nach einem der Ansprüche 1 bis 5, wobei die Nachweisantikörper, Zweitantikörper und markierten Antikörper monoclonale Antikörper oder von monoclonalen Antikörpern abgeleitet sind.

7. Verfahren nach einem der Ansprüche 2 bis 6, wobei die Markierung ein enzymatisches Molekül ist, das bei Behandlung einen Farbwechsel erzeugt, um einen sichtbaren Beweis für die Gegenwart der Markierung und Kolonien der ausgewählten Bakterien auf der Membran zu liefern.

8. Verfahren nach Anspruch 7, wobei das enzymatische Molekül Peroxidase, alkalische Phosphatase, ·-Galactosidase, ·-Glucuronidase, ·-Glucosidase, ·-Glucosidase, ·-Mannosidase, Galactose-Oxidase, Glucose-Oxidase oder Hexokinase ist.

9. Verfahren nach Anspruch 8, wobei die Membran mit einem chromogenen Substrat für das enzymatische Molekül behandelt wird.

10. Verfahren nach einem der vorstehenden Ansprüche, wobei die Kolonie-Hub-Membran Nitrocellulose oder Nylon umfaßt.

11. Verfahren nach einem der vorstehenden Ansprüche, wobei nach dem Inkontaktbringen mit den Bakterienkolonien die Membran mit einem Fixierungsmittel behandelt wird, um die Kolonien auf der Membran zu fixieren.

12. Verfahren nach einem der vorstehenden Ansprüche, wobei die Bakterien ausgewählt sind aus Listeria, Campylobacter, Escherichia coli, Salmonella, Clostridium, Shigella, Staphylococcus, Vibrio, Yersinia, Plesiomonas strigelloides, Bacillus und Aeromonas.

13. Verfahren nach Anspruch 12, wobei die ausgewählten Bakterien Listerien sind.

14. Verfahren nach Anspruch 13, wobei die ausgewählten Bakterien pathogene Listerienstämme sind.

## Revendications

1. Procédé pour détecter la présence de bactéries qui comprend :
(a) le mélange d'un échantillon dans lequel la présence de bactéries choisies est à tester, avec des billes magnétiques sur lesquelles sont immobilisées des premiers anticorps dirigés contre lesdites bactéries choisies ;
(b) l'incubation dudit échantillon et desdites billes magnétiques sur lesquelles sont immobilisées lesdits anticorps, pour capturer ainsi des cellules desdites bactéries choisies dudit échantillon ;
(c) l'exposition à un champ magnétique desdites billes magnétiques auxquelles sont liés lesdits premiers anticorps et cellules bactériennes capturées, pour séparer ainsi lesdites billes magnétiques dudit échantillon ;
(d) la culture desdites billes magnétiques et cellules bactériennes capturées, sur un support solide, pour former des colonies desdites bactéries ;
(e) la mise en contact desdites colonies avec une membrane de transfert de colonies, ce par quoi de la matière de colonie venant desdites colonies adhère à ladite membrane ; et
(f) la mise en contact de ladite membrane avec des anticorps détecteurs qui se lient à ladite matière de colonie desdites bactéries choisies, et la présence dudit anticorps détecteur sur ladite membrane est détectée pour offrir une preuve visuelle de la présence, sur ladite membrane, de matière de colonie venant desdites bactéries choisies.

2. Procédé selon la revendication 1, dans lequel ladite membrane sur laquelle adhère la matière de colonie est mise en contact avec des seconds anticorps spécifiques desdites bactéries choisies, ce par quoi lesdits seconds anticorps se tient à ladite matière de colonie venant desdites colonies desdites bactéries choisies ; ladite membrane est par la suite mise en contact avec des anticorps marqués spécifiques desdits seconds anticorps, et la présence dudit marqueur sur ladite membrane est détectée pour détecter ainsi la présence desdites bactéries choisies dans ledit échantillon.

3. Procédé selon la revendication 1, dans lequel ladite membrane est mise en contact avec des anticorps marqués spécifiques desdites bactéries choisies, ce par quoi lesdits anticorps marqués se lient à ladite matière de colonie venant desdites bactéries choisies, et la présence dudit marqueur sur ladite membrane est détectée pour détecter ainsi la présence desdites bactéries choisies dans ledit échantillon.

4. Procédé selon la revendication 2 ou 3, dans lequel ledit marqueur est choisi parmi des molécules fluorescentes, radioactives, chimioluminescentes, bioluminescentes, et des molécules de substrat enzymatique.

5. Procédé selon l'une quelconque des revendications précédentes, dans lequel lesdits premiers anticorps sont des anticorps polyclonaux dirigés contre lesdites bactéries choisies.

6. Procédé selon l'une quelconque des revendications 1 à 5, dans lequel lesdits anticorps détecteurs, seconds anticorps et anticorps marqués sont des anticorps monoclonaux ou dérivent d'anticorps monoclonaux.

7. Procédé selon l'une quelconque des revendications 2 à 6, dans lequel ledit marqueur est une molécule enzymatique, qui lorsqu'elle est traitée, produit un changement de couleur pour offrir une preuve visuelle de la présence sur ladite membrane dudit marqueur et de colonies desdites bactéries choisies.

8. Procédé selon la revendication 7, dans lequel ladite molécule enzymatique est la peroxydase, la phosphatase alcaline, la β-galactosidase, la β-glucuronidase, l'α-glucosidase, la β-glucosidase, l'α-mannosidase, la galactose oxydase, la glucose oxydase, ou l'hexokinase.

9. Procédé selon la revendication 8, dans lequel ladite membrane est traitée avec une substance chromogène pour ladite molécule enzymatique.

10. Procédé selon l'une quelconque des revendications précédentes, dans lequel ladite membrane de transfert de colonies se compose de nitrocellulose ou de nylon.

11. Procédé selon l'une quelconque des revendications précédentes, dans lequel après mise en contact avec lesdites colonies bactériennes, ladite membrane est traitée avec un agent de fixation pour fixer lesdites colonies sur ladite membrane.

12. Procédé selon l'une quelconque des revendications précédentes, dans lequel lesdites bactéries sont choisies parmi les Listeria, Campylobacter, Escherichia coli, Salmonella, Clostridia, Shigella, Staphylococci, Vibrio, Yersinia, Plesiomonas strigelloides, Bacilli et Aeromonas.

13. Procédé selon la revendication 12, dans lequel lesdites bactéries choisies sont des Listeria.

14. Procédé selon la revendication 13, dans lequel lesdites bactéries choisies sont des souches pathogènes de Listeria.
